(19) Europäisches Patentamt
European Patent Office
Office européen des brevets

(11) **EP 4 410 322 A1**

(12) **EUROPEAN PATENT APPLICATION**

(43) Date of publication:
**07.08.2024 Bulletin 2024/32**

(21) Application number: **23154695.3**

(22) Date of filing: **02.02.2023**

(51) International Patent Classification (IPC):
*A61L 26/00* (2006.01)     *C08G 18/10* (2006.01)
*C08G 18/16* (2006.01)     *C08G 18/48* (2006.01)
*C08G 18/50* (2006.01)     *C08G 18/73* (2006.01)

(52) Cooperative Patent Classification (CPC):
(C-Sets available)
**C08G 18/10; A61L 31/06; A61L 31/145;
A61L 31/16; C08G 18/168; C08G 18/4837;
C08G 18/4887; C08G 18/5024; C08G 18/73;**
C08G 2210/00; C08G 2230/00     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Covestro Deutschland AG
51373 Leverkusen (DE)**

(72) Inventor: **The designation of the inventor has not
yet been filed**

(74) Representative: **Levpat
c/o Covestro AG
Gebäude K12
51365 Leverkusen (DE)**

(54) **METHOD OF MANUFACTURING A HYDROGEL USEFUL FOR PREVENTING POST-SURGICAL ADHESIONS**

(57)     A method of manufacturing a hydrogel comprises reacting:

A) An isocyanate-terminated prepolymer obtained by the reaction of at least:

A1) A diisocyanate with a molar mass of 140 to 278 g/mol;

A2) A polyalkylene oxide with an ethylene oxide content of $\geq$ 50 weight-%, calculated from the total weight of the polyalkylene oxide;

B) A polyamine;

C) Water.

The polyalkylene oxide A2) and/or the polyamine B) further comprise ester groups and the average OH functionality of polyalkylene oxide A2) is greater than 2 and/or the average combined primary and secondary amine functionality of polyamine B ) is greater than 2.

**EP 4 410 322 A1**

(52) Cooperative Patent Classification (CPC): (Cont.)

C-Sets
**A61L 31/06, C08L 75/08;**
**C08G 18/10, C08G 18/3228;**
**C08G 18/10, C08G 18/50**

**Description**

The present invention relates to a method of manufacturing a hydrogel comprising reacting: A) An isocyanate-terminated prepolymer obtained by the reaction of at least: A1) A diisocyanate with a molar mass of 140 to 278 g/mol; A2) A polyalkylene oxide with an ethylene oxide content of ≥ 50 weight-%, calculated from the total weight of the polyalkylene oxide; B) A polyamine and C) Water. The invention also relates to a hydrogel obtainable by such a method, the use of such a hydrogel in surgery, as a post-surgical adhesion barrier and to a device for discharging a mixture from which the hydrogel is formed.

Adhesions are among the most frequent complications after surgical interventions in the abdominal and pelvic regions. Adhesions are fibrous bands which generally form within the first seven days after an operation, in the course of the healing process. They cause tissues and organs which are normally separated from one another to grow together, which can give rise to a multiplicity of complications such as, for example, chronic pain, infertility or a lifethreatening intestinal occlusion. Products able to reduce the formation of adhesions have been developed in recent years to avoid such complications.

Hydrogels have been used as adhesion barriers as well as other materials. Hydrogels are hydrophilic crosslinked polymers that do not dissolve in it. Crosslinks can be formed by covalent chemical bonds or non-covalent interactions such as electrostatic, hydrophobic or dipole-dipole interactions between individual segments of polymer chains, building a three-dimensional network. These networks swell in water up to an equilibrium volume with substantial shape retention. Desired properties of hydrogels are specifically targetable via the choice of monomers used for polymer construction, the type of crosslinking and the crosslink density.

DE 20 2018 004305 U1 discloses a polyurethane hydrogel obtained by the reaction of at least the components a) a hydrophilic aliphatic and/or cycloaliphatic polyisocyanate prepolymer with a monomeric diisocyanate content of less than 1 weight-% with b) a compound having at least one amino group in the presence of c) water. Compound b) has a primary amino group and a carboxylic acid group. Salts of weak acids and zwitterions are included as well. The polyurethane hydrogel may be part of a wound dressing.

US 2012/244107 A1 relates to a hydrogel based on polyurethane or polyurethaneurea, having hydrolyzable functional groups in the polymer chain and obtained by reaction of A) a polyisocyanate prepolymer having hydrolyzable groups in the polymer chain, B) water, C) optionally hydroxyl-amino compounds having at least one tertiary amino group and at least three hydroxyl groups, D) optionally catalysts, and E) optionally auxiliary and addition agents. Said polyisocyanate prepolymer A) is obtained by reaction of A1) a polyisocyanate with A2) a polyol having hydrolyzable groups in the polymer chain. Said polyol A2) comprises polyesters and/or polyetheresters that are liquid at room temperature and have a DIN 53019 shear viscosity at 23° C in the range of 200 to 8000 mPas. Also disclosed is an adhesion barrier comprising the claimed hydrogel.

US 4,795,764 concerns a poly(oxyalkylene) poly(aliphatic isocyanate) prepolymer and polyurea polymer derived therefrom by reaction with polyamine. Discussed is a solution comprising polyamine reactant, low moisture sensitive poly(oxyalkylene) poly(aliphatic isocyanate) prepolymer the oxyalkylene portion of which contains sufficient oxyethylene units to render the prepolymer hydrophilic and water-soluble, and a water-soluble or -dispersible organic solvent which is free of active hydrogen atoms and in which said prepolymer is dissolved, the isocyanate moieties of said prepolymer being reactive with said polyamine. Further disclosed is an aqueous solution comprising the aforementioned solution and water as the major component of the aqueous solution.

WO 2003/050276 A1 discloses a polyurethane-hydrogel composition having an immobilized biologic, said composition being prepared from a process comprising the steps of (a) admixing at least one prepolymer and at least one water-soluble crosslinker in aqueous solvent and in the substantial absence of organic solvent to form a polyurethane-hydrogel mixture, said prepolymer being prepared from at least one water-soluble polyol and at least one isocyanate; and (b) contacting said mixture with a biologic to immobilize the biologic in said mixture to form a composition having an immobilized biologic, wherein said composition is substantially polymerized, is transparent, and has an effective number-average molecular weight between crosslinks.

US 2013/060216 A1 relates to a hydrogel matrix with improved adhesive characteristics. US 2013/204217 A1 relates to a hydrogel matrix having increased absorption capacity for liquids.

The biodegradable hydrogels in the art for use as adhesion barriers suffer from the drawback that some of the components needed for their formation have high viscosities. This limits their application to body surfaces via spraying.

The present invention has the object of providing a hydrogel which is suitable as a post-surgical adhesion barrier having enhanced biodegradability and which can be applied by spraying. This object has been achieved by a method according to claim 1 and a hydrogel according to claim 12. The invention also relates to a hydrogel for use according to claims 13 and 14 and a device according to claim 15. Advantageous embodiments are the subject of the dependent claims. They can be combined freely unless the context clearly indicates otherwise. Accordingly, a method of manufacturing a hydrogel comprises reacting the components:

A) An isocyanate-terminated prepolymer obtained by reacting a mixture comprising: A1) a diisocyanate with a molar weight of 140 to 278 g/mol; A2) a polyalkylene oxide with an ethylene oxide content of $\geq$ 50 weight-%, calculated from the total weight of the polyalkylene oxide; B) A polyamine having a molecular weight of $\geq$ 200 g/mol and C) water.

Examples for suitable polyisocyanates A1) include methylene diphenyl diisocyanate (MDI), methylene dicyclohexyl diisocyanate (H12-MDI), polymeric MDI, toluylene diisocyanate (TDI), xylylene diisocyanate (XDI), pentamethylene diisocyanate (PDI), hexamethylene diisocyanate (HDI), isophorone diisocyanate (IPDI) and a mixture comprising at least two of the aforementioned polyisocyanates.

The polyalkylene oxide A2) and/or the polyamine B) further comprise ester groups, expressed as -C(=O)-O-, in an amount of > 0.51 weight-%, based on the total weight of polyalkylene oxide A2) or polyamine B), respectively. It is preferred that the polyalkylene oxide A2) comprises ester groups of > 0.51 weight-%, based on the total weight of polyalkylene oxide A2), while the polyamine B) does not comprise ester groups. Preferably the ester group content in A2) and/or B is $\leq$ 5.1 weight-%. Further preferred ranges are > 0.51 weight-% to $\leq$ 5 weight-%, > 0.51 weight-% to $\leq$ 4.5 weight-% or $\geq$ 1 weight% to $\leq$ 4.5 weight-%, more preferred $\geq$ 1.2 weight-% to $\leq$ 3 weight-%.

Ester groups, which include diester block in the context of the present invention, may be introduced into the polyalkylene A2) by adding a cyclic ester dimer and/or polyester to a reaction mixture comprising a starter and alkylene oxide(s). The same considerations apply to the synthesis of ester group-containing polyamines B).

Examples for suitable cyclic ester dimers include dilactide (providing "lactide groups") and glycolide (providing "glycolide groups"). Examples for suitable polyesters include polylactic acid and polyglycolic acid. Mixtures of two or more of the aforementioned compounds can also be used.

The ester group contents for polyether polyols may be calculated by the following equation:

$$Ester\ content_{Polyol} = \frac{\dfrac{m_{cyclic\ ester} * 44 * f}{MW_{cyclic\ ester}}}{m_{EO} + m_{PO} + m_{Dilactid} + m_{Starter}} * 100 \qquad (1.1)$$

| | |
|---|---|
| $m_{cyclic\ ester}$= Total mass of added dilactide | $MW_{cyclic\ ester}$= molecular weight of cyclic ester (for all conducted experiments = 144 g/mol) |
| 44=molar mass of -O-C=O | $m_{EO}$; $m_{PO}$; $m_{Dilactid}$; $m_{Starter}$= total mass of EO/PO/ dilactide/starter |
| $f$= factor for the amount of ester units per cyclic ester (for all conducted experiments =2) | 100=factor for percentage |

The ester group contents for prepolymers may be calculated by the following equation:

$$Ester\ content_{Prepolymer} \qquad (1.2)$$

$$= \frac{\dfrac{m_{polyol} * ester\ content_{polyol}}{100}}{(\dfrac{m_{polyol}}{eq.wght.polyol} * MW_{Diisocyanate}) + m_{polyol}} * 100$$

| | |
|---|---|
| $m_{polyol}$= Total mass of added polyol | eq. wght. polyol= equivalent weight of the polyol |
| ester content$_{polyol}$= calculated as in eq. 1.1 | $MW_{Diisocyanate}$= molar mass of used diisocyanate (for all conducted experiments =168.2 g/mol) |

The polyamine B) preferably has a molecular weight of $\geq$ 300 g/mol and $\leq$ 10000 g/mol as determined by amine number determination according to DIN EN ISO 1877-2. Molecular weight may be calculated as described in equation 1.3, wherein F is the NH$_2$ functionality, AN is the determined amine number according to DIN EN ISO 1877-2 and 56100 as molecular weight of potassium hydroxide multiplied with 100.

$$Molecular\ weight = \frac{F \cdot 56100}{AN} \qquad (1.3)$$

The average OH functionality of polyalkylene oxide A2) is greater than 2 and/or the average combined primary and secondary amine functionality of polyamine B) is greater than 2. Possible combinations include a trifunctional polyalkylene oxide A2) and a difunctional polyamine B), a trifunctional polyalkylene oxide A2) and a trifunctional polyamine B) and a difunctional polyalkylene oxide A2) and a trifunctional polyamine B).

The combined components A) and B) are present in an amount of > 11.5 weight-% ("solids content"), based on the total weight of components A), B) and C). Preferred is an amount of > 11.5 weight-% to ≤ 50 weight-%, more preferred > 13 weight-% to ≤ 40 weight-%, even more preferred ≥ 13.2 weight-% to ≤ 20 weight-%.

It has been found that the hydrogels obtainable by the method according to the invention have the combined properties of a useful biodegradability profile, an adhesion to internal organs and a usefully fast time for their formation. In preferred embodiments it has been found that the biodegradability, determined via loss of mechanical properties in accordance with the method laid out in the experimental section of this disclosure, is > 6 days to ≤ 28 days. In preferred embodiments it has been found that the adhesion to internal organs is good or very good, determined in accordance with the method laid out in the experimental section. In preferred embodiments it has been found that the formation time for the hydrogels, determined in accordance with the method laid out in the experimental section, is ≤ 60 seconds.

The reaction mixture for obtaining the hydrogels and the thus obtained hydrogels may be free from urethane group formation catalysts.

In one embodiment the NCO/NH molar ratio between prepolymer A) and polyamine B) is > 1.0 to ≤ 1.4. Preferred is a ratio of ≥ 1.1 to ≤ 1.3.

In another embodiment the prepolymer A) and/or the polyamine B) are provided as aqueous solutions. The concentrations of prepolymer A) and/or polyamine B), expressed as solids contents, may independently be in a range of ≥ 5 weight-% to ≤ 20 weight-%, based on the total weight of the solution.

In another embodiment the aqueous solution of prepolymer A) and/or the aqueous solution of polyamine B) have a viscosity of ≤ 50 mPas at 23 °C as determined according to DIN EN ISO 3219 Preferred are viscosities of ≥ 1 mPas to ≤ 20 mPas, more preferred ≥ 2 mPas to ≤ 10 mPas.

In another embodiment the water C) comprises an acid or a pH buffer. This has the advantage that a rise in pH on the surface of, for example, an internal organ to which is in contact with the hydrogel is limited. Preferably the pH does not rise over 8 after 30 minutes following the application of the hydrogel. Buffer concentrations are preferred in the range of ≥ 0% to ≤ 50% H$^+$ with regard to free amine groups calculated according to equation 1.4. Preferably $f_{buffering\ grade\ amine}$ is in the range of 0 to 0.5, more preferred in the range of 0 to 0.4 and even more preferred 0 to 0.25.

$$f_{buffering\ grade\ amine} = \frac{V_{solution} * M_{acidic\ component}}{\dfrac{V_{solution} * \delta_{solution} * \dfrac{solid\ content_{amine}}{100}}{eq.\ weight_{amine}}} \qquad (1.4)$$

$M_{acidic\ component}$ = Mol/l of acidic component

$V_{solution}$ = Volume of amine component

$\delta_{solution}$ = density of the solution (for all conducted experiments =1)

$f_{buffering\ grade\ amine}$ = grade of buffered free amine groups in the range of 0.0-1.0

$eq.\ weight_{amine}$ = Equivalent weight amine as determined

100 = factor for percentage

In another embodiment the diisocyanate A1) is an aliphatic diisocyanate. Preferred examples include pentamethylene diisocyanate, hexamethylene diisocyanate, isophorone diisocyanate and H12-MDI.

In another embodiment the prepolymer A) has an NCO group content of ≥ 1 weight-% to ≤ 10 weight-% as determined according to DIN EN ISO 11909. Preferred are ≥ 2 weight-% to ≤ 4 weight-%.

In another embodiment the polyamine B) is an amino group-terminated polyether. Such polyether amines may comprise primary amino groups attached to polyether backbones, typically based on propylene oxide, ethylene oxide or a mixture of both oxides. Commercially available examples include those found in the Jeffamine product families. Preferred are trimethylolpropane- (TMP) or glycerol- (glycerin) started amino group-terminated polyoxpropylenes, commercial examples of which can be found in the Jeffamine T series. Other preferred polyether amines are polyether diamines with more polyoxypropylene groups than polyoxyethylene groups in the backbone, commercial examples of which can be found in the Jeffamine ED series. Specific examples are Jeffamine ED2003 and Jeffamine T403 as described in the experimental section of this disclosure.

In another embodiment the equivalent weights of the prepolymer A) and the polyamine B) have a ratio of $\geq$ 0.25 to $\leq$ 4. Preferably the equivalent weight ratios are $\geq$ 0.5 to $\leq$ 2, more preferred $\geq$ 1.5 to $\leq$ 1.7. This facilitates spraying applications of aqueous solutions of prepolymer A) and polyamine B).

In another embodiment aqueous solutions of prepolymer A) and polyamine B) are mixed in a mixing or spraying head and the resulting mixture is applied onto a target surface, thereby forming the hydrogel on the target surface. The target surface may be an internal organ during surgery.

In another embodiment the method further comprises adding as component D) an active pharmaceutical ingredient to a precursor of the hydrogel and/or to the hydrogel itself.

Suitable active pharmaceutical ingredients include:

1. Anti-infectives, such as antibiotics, including penicillin, tetracycline, chlortetracycline bacitracin, nystatin, streptomycin, neomycin, polymyxin, gramicidin, oxytetracycline, chloramphenicol, and erythromycin; sulfonamides, including sulfacetamide, sulfamethazine, sulfadiazine, sulfamerazine, sulfamethizole and sulfisoxazole; antivirals, including idoxuridine; and other anti-infectives including nitrofurazone and sodium propionate.

2. Anti-allergenics such as antazoline, methapyrilene, chlorpheniramine, pyrilamine and prophenpyridamine.

3. Anti-inflammatories such as hydrocortisone, cortisone, dexamethasone 21-phosphate, fluocinolone, triamcinolone, medrysone, prednisolone, prednisolone 21-phosphate, and prednisolone acetate.

4. Decongestants such as phenylephrine, naphazoline, and tetrahydrazoline.

5. Miotics and antichlolinesterases such as pilocarpine, eserine salicylate, carbachol, diisopropyl fluorophosphate, phospholine iodide, demecarium bromide, physostigmin and neostigmin.

6. Mydriatics such as atropine sulfate, cyclopentolate, homatropine, scopolamine, tropicamide, eucatropine, and hydroxyamphetamine.

7. Sympathomimetics such as epinephrine.

8. Neuroleptics such as reserpine and chlorpromazine.

9. Estrogens such as estrone, 17$\beta$-estradiol, ethinyl estradiol, and diethyl stilbesterol.

10. Progestational agents such as progesterone, 19-norprogesterone, norethindrone, megestrol, melengestrol, chlormadinone, ethisterone, medroxyprogesterone, norethynodrel and 17a-hydroxy-progesterone.

11. Humoral agents such as the prostaglandins, for example, PGE1, PGE2, and PGF2.

12. Antipyretics such as ibuprofen, acetylsalicylic acid, sodium salicylate, and salicylamide.

13. Antispasmodics such as atropine, methantheline, papaverine, and methscopolamine bromide.

14. Cardioactive agents such as benzydroflumethiazide, flumethiazide, chlorothiazide, and aminotrate.

One type of preferred pharmaceutical compounds is a progestogen or a drug having a progestogenic activity. In particular the compound may be selected from the group of progesterone and its derivatives, cyproterone acetate, desogestrel, etonogestrel, levonorgestrel, lynestrenol, medroxyprogesterone acetate, norethisterone, norethisterone acetate, norgestimate, drospirenone, gestodene, 19-nor-17-hydroxy progesterone esters, 17$\alpha$-ethinyltestosterone and derivatives thereof, 17$\alpha$-ethinyl-19-nor-testosterone and derivatives thereof, ethynodiol diacetate, dydrogesterone, norethynodrel, allylestrenol, medrogestone, norgestrienone, ethisterone, dl-norgestrel or a mixture of at least two of the aforementioned compounds.

Another type of preferred pharmaceutical compound is a drug capable of preventing or suppressing endometrial bleeding. In particular the compound may be selected from the group of prostaglandin synthesis inhibitors, NSAIDs, inhibitors of leukotriene, oxytocin antagonists, pancreatic trypsin inhibitors, COX-inhibitors, antifibrinolytic drugs, estrogens, antiestrogens, aromatase inhibitors, cytokine inhibitors, glucocorticoids, progestogens with pronounced glucocorticoid acticity, danazol, gestrinone, angiogenesis inhibitors or a mixture of at least two of the aforementioned compounds.

It is possible to combine the progestogen or a drug having a progestogenic activity with the drug capable of preventing or suppressing endometrial bleeding. Particularly preferred is the combination of levonorgestrel with tranexamic acid, mefenamic acid, danazol or an angiogenesis inhibitor. This is useful in an intrauterine device.

Another type of preferred pharmaceutical compound is a calcium channel blocker, in particular nimodipine or nifedipine.

Another type of preferred pharmaceutical compound is a cytostatic agent such as cisplatin, rituximab, bevacizumab, trastuzumab, imatinib, lenalidomide, pemetrexed, bortezomib, cetuximab, leuprolein or abiraterone.

Lastly, opioid analgetics and non-opioid analgetics are also a type of preferred pharmaceutical compound.

The pharmaceutical compounds (drugs) can be in different forms, such as uncharged molecules, components of molecular complexes, or non-irritating, pharmacologically acceptable salts such as hydrochloride, hydrobromide, sulphate, phosphate, nitrate, borate, acetate, maleate, tartrate, salicylate, etc. For acidic drugs, salts of metals, amines, or organic cations (e.g., quaternary ammonium) can be employed. Furthermore, simple derivatives of the drugs (such as ethers, esters, amides, etc.) which have desirable retention and release characteristics but which are easily hydrolyzed by body pH, enzymes, etc., can be employed.

The hydrogel according to the invention can be used as a delayed-release formulation for the pharmaceutical compound, pesticide, herbicide, pheromone or combination of at least two of the aforementioned compounds distributed within the hydrogel.

In its use as a delayed release formulation the hydrogel can be viewed as a drug delivery device or system. Drug delivery systems of the invention can take a wide variety of shapes and forms for administering the drugs at controlled rates to different areas of the body. Thus, the invention includes external and internal drug-delivery systems such as skin patches, sublingual or buccal tablets, peroral dosage forms, implants for releasing a drug in the tissues of a living organism, pessaries, prosthesis, artificial glands, vaginal or rectal suppositories, cervical rings, troches, drug-dispensing intrauterine devices and ocular inserts.

Another aspect of the invention is a hydrogel obtainable by a method according to the present invention.

Another aspect of the invention is a hydrogel obtainable by a method according to the present invention for use in a method of surgery.

Another aspect of the invention is a hydrogel obtainable by a method according to the present invention for use as a post-surgical adhesion barrier. The prevention of intraabdominal adhesions is expressly contemplated.

Another aspect of the invention is a device comprising a first storage volume, a second storage volume and a mixer in fluid communication with the first and second storage volume, wherein the mixer is configured to mix the contents of the first and second storage volume and to discharge the resulting mixture from the device, wherein the first storage volume comprises the prepolymer A) as recited in connection with the method according to the present invention, the second storage volume comprises the polyamine B) as recited in connection with the method according to the present invention. The first and/or second storage volumes may further comprise water. It is also possible that the device comprises a third storage volume which comprises water. The water may be added to the prepolymer A) and/or polyamine B) prior to their mixing.

In one embodiment of the device the equivalent weights of the prepolymer A) and the polyamine B) have a ratio of $\geq 0.25$ to $\leq 4$. Preferably the equivalent weight ratios are $\geq 0.5$ to $\leq 2$, more preferred $\geq 1.5$ to $\leq 1.7$. This facilitates spraying applications of aqueous solutions of prepolymer A) and polyamine B).

The present invention shall be further described with reference to the following examples without wishing to be limited by them.

**Experimental section:**

Apparatus and Analytical Methods Used:

pH-Meter: Knick Portamess 911 pH,

Viscosity determination: DIN EN ISO 3219

Residual monomer content determination: DIN EN ISO 10283

Hydroxyl number determination: DIN EN ISO 4629-2

Acidic number determination: DIN EN ISO 2114

Amine number determination: DIN EN ISO 1877-2

NCO content determination: DIN EN 1242

Equivalent weight determination: The equivalent weight (eq. weight) was determined according to the following equation:

$$eq.\,weight = \frac{42 * 100}{NCO\ content\ \%}$$

**Table 1: Raw materials used for the experiments**

| Name | CAS | Purity | Supplier |
|---|---|---|---|
| **Jeffamines** | | | |
| Jeffamine ED2003 | 65605-36-9 | n.d. | Huntsman |
| Jeffamine T403 | 39423-51-3 | n.d. | Huntsman |
| **Diisocyanates** | | | |
| Hexamethylene diisocyanate | 822-06-0 | n.d. | Covestro AG |
| **Starters and monomers for polyol synthesis** | | | |
| Polyether 1 | - | n.d. | Covestro AG |
| Polyether 2 | - | n.d. | Covestro AG |
| Ethylene oxide | 75-21-8 | n.d. | GHC Gerling Holz |
| Propylene oxide | 75-56-9 | n.d. | Chemogas |
| Dilactide | 95-96-5 | >99% | Sigma-Aldrich |
| **Polyols** | | | |
| Polyether 3 | - | n.d. | Covestro AG |
| **Catalysts** | | | |
| dibutyl phosphate | 107-66-4 | ≥ 97.0% | Sigma-Aldrich |
| **Buffer agents** | | | |
| HC10.1M | 7647-01-0 | n.d. | Honeywell Fluka |
| Sodium acetate | 127-09-3 | ACS reagent, ≥ 99.0% | Sigma-Aldrich |
| Acetic acid, glacial | 64-19-7 | ACS reagent, ≥ 99.7% | Sigma-Aldrich |
| Sodium chloride | 7647-14-5 | +80 mesh, ≥ 98% | Sigma-Aldrich |
| Potassium chloride | 7447-40-7 | ACS reagent, 99.0-100.5% | Sigma-Aldrich |
| Potassium phosphate monobasic, anhydrous | 7778-77-0 | ACS reagent, ≥ 99.0% | Sigma-Aldrich |
| di-Sodium hydrogen phosphate heptahydrate | 7782-85-6 | EMPROVE® EXPERT, DAC, USP | Merck |
| Sodium dihydrogen phosphate monohydrate | 10049-21-5 | EMPROVE® EXPERT, BP, USP | Merck |

Jeffamine ED2003 was an O,O'-bis(2-aminopropyl) polypropylene glycol-*block*-polyethylene glycol-*block*-polypropylene glycol having a molecular weight of ca. 2000 g/mol and the idealized formula $CH_3CH(NH_2)CH_2[OCH(CH_3)CH_2]_1(OCH_2CH_2)_m[OCH_2CH(CH_3)]_nNH_2$ with m being ca. 39 and (1+n) being ca. 6.

Jeffamine T403 was a trimethylolpropane tris[poly(propylene glycol), amine terminated] ether having a molecular weight of ca. 440 g/mol and the idealized formula $C_2H_5C[CH_2[OCH_2CH(CH_3)]_nNH_2]_3$ with n being 5 to 6.

**Preparation of amine solutions**

For the preparation of the amine solution, which was used for the preparation of the hydrogels, either Jeffamine ED2003, predominantly based on PEG, functionality=2, equivalent weight=1021 g/eq. (Huntsman) or Jeffamine T403, predominantly based on PEG, functionality=3, equivalent weight=81 g/eq. (Huntsman), were used. Solutions were prepared in aqueous media using deionized $H_2O$ and/or buffers or aqueous acid like phosphate (monobasic/dibasic), acetic acid/acetate buffer, HCl. Composition of the prepared solutions is stated in table 2.

**Preparation of hydrogels:**

Two cylinders were used for the application. One with a volume of 25 ml (Isocyanate component) and one with a volume of 12.5 ml (amine component). For the mixing process a static mixer MAH 4.0-17S from AdChem was used. Both components were dispensed in a constant volume ratio of 2:1 (isocyanate:amine) within 25 seconds.

**Determination of pH-values**

pH-measurements were conducted by spraying both components into a beaker with a pH electrode inside. pH-measurements were conducted for 30 minutes.

**Determination of adhesion on organs:**

A kidney (pig) preheated to 37 °C was used as a substrate for the application. Hydrogels were prepared as described above. The following rating system may be used to evaluate the adhesion of each formulation to the kidney.

| Rating Score | Adhesion Properties |
|---|---|
| 1 | No adhesion |
| 2 | Very poor adhesion (hydrogel can be removed without applying mechanical force, e.g., by movement of the kidney) |
| 3 | Poor adhesion (hydrogel can be removed by applying light mechanical force, like wiping over the surface with a moistened gloves) |
| 4 | Good adhesion (hydrogel can be removed by applying stronger mechanical force, like pulling or rubbing) |
| 5 | Very good adhesion (hydrogel cannot be removed as a whole by applying strong mechanical force, like pulling or rubbing) |

**Determination of biodegradability via loss of mechanical properties:**

For the determination of the biodegradation rate hydrogels were prepared as described above but dispensed into 3.9 mL PE molds (thick end of 2 ml type pipettes, $V_{total}$=5.9 ml by VITLAB®). After curing for 5 min they were weighed and transferred into a 25 mL glass bottle. 22 ml PBS-solution (pH=7.33) were added and the glass bottles were stored at 37 °C in an incubator with shaking speed of 60 rpm. Every 24 h the hydrogel samples were taken out of the buffer solution to record the weight. Afterwards, the old buffer solution was replaced with fresh buffer solution and the samples were again stored in the incubator. This procedure was repeated every 24 hours and the samples were incubated until they were fully dissolved in the medium. The point at which the hydrogels could not be removed from the glass bottles without disintegration into smaller gel particles is described as "Loss of mechanical properties" (table 4). Without wishing to be bound by theory it is assumed that there would be no barrier effect of the hydrogel anymore, so this point in time is regarded as an end point.

**Determination of curing time**

To determine the curing time of each hydrogel formulation, the mixtures were sprayed on a vertical release paper and the time the mixed solutions needed for the gelation process was measured. The curing time was defined as the point where no more liquids ran from the release paper and the droplets began to form a hydrogel according to visual observation.

**Spray experiment example:**

Application example (cf. example 13):

Provision of the amine component:

For a preparation of 12.5 ml of amine solution which was used in a ratio of 1/1.2 ($NH_2$/NCO groups), 1.82 g of Jeffamine ED2003 were weighed into a flask and added up to 12.50 g with aqueous HCl (0.0260M). Afterwards, the solution was stirred and then filled into a cylinder of a spray applicator.

Provision of the isocyanate component:

3.75 g of prepolymer 4 were weighed into a mixing cup and filled up to 25.00 g with demineralized $H_2O$. The solution was mixed for 30 seconds. Afterwards the solution was filled into a cylinder of a spray applicator and the spray experiments were conducted immediately (within 15 seconds).

**Polyol synthesis**

**Polyol 1**

1645.00 g of a glycerin (F=3) started polyoxypropylene triol (Polyether 1; Covestro Charge 1712041028) with an OH-number of 233 mg KOH/g, 83.50 g of dilactide (Aldrich, LOT BCBV8611) and 2.00 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C. 2215.00 g of propylene oxide (Chemogas) and 6058.00 g of ethylene oxide (GHC Gerling Holz) were dosed within 135 min. to the reaction mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.

Ester group content expressed as -C(=O)-O-: 0.51 wt.-%

Ethylene oxide content: 60.57 wt.-%

OH number: 39.90 mg KOH/g

Acid number: 0.01 mg KOH/g

Viscosity: 1010 mPa*s, 25 °C

PO/EO/dilactide amount ratios: 26.5%/72.5%/1%

**Polyol 2**

1645.00 g of a glycerin (F=3) started polyoxypropylene triol (Polyether 1; Covestro Charge 1712041028) with an OH-number of 233 mg KOH/g, 209.00 g of dilactide (Aldrich, LOT BCBV8611) and 2.00 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C. 2069.00 g of propylene oxide (Chemogas) and 6058.00 g of ethylene oxide (GHC Gerling Holz) were dosed within 135 min. to the rection mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.

Ester group content expressed as -C(=O)-O-: 1.28 wt.-%

Ethylene oxide content: 60.70 wt.-%

OH number: 39.7 mg KOH/g

Acid number: 0.01 mg KOH/g

Viscosity: 1040 mPa*s, 25 °C

PO/EO/dilactide amount ratios: 25.0%/72.5%/2.5%

**Polyol 3**

1194.70 g of a glycerin (F=3) started polyoxypropylene triol (Polyether 1; Covestro Charge 1712041028) with an OH-number of 233 mg KOH/g, 300.70 g of dilactide (Aldrich, LOT BCBV8611) and 0.75 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C and the pressure to 2.21 bar with nitrogen. 1353.40 g of propylene oxide (Chemogas) and 4361.00 g of ethylene oxide (GHC Gerling Holz) were dosed within 135 min. to the rection mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.

Ester group content expressed as -C(=O)-O- 2.56 wt.-%

Ethylene oxide content: 60.48 wt.-%

OH number: 38.5 mg KOH/g

Acid number: 0.02 mg KOH/g

Viscosity: 1145 mPa*s, 25 °C

PO/EO/dilactide amount ratios (wt.%): 22.5%/72.5%/5%

**Polyol 4**

1643.00 g of a glycerin (F=3) started polyoxypropylene triol (Polyether 1; Covestro Charge 1712041028) with an OH-number of 233 mg KOH/g, 833.0 g of dilactide (Aldrich, LOT BCBV8611) and 2.0 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C. 1450.00 g of propylene oxide (Chemogas) and 6058.00 g of ethylene oxide (GHC Gerling Holz) were dosed within 135 min. to the rection mixture and were reacted for further 45 min at 130 °C. Afterwards the reaction was cooled to 90 °C and volatile compounds were distilled off for 30 min. at <0.01 bar. The reaction mixture was stabilized with 6.00 g of Irganox 1076.

Ester group content expressed as -C(=O)-O-: 5.09 wt.-%

Ethylene oxide content: 60.68 wt.-%

OH number: 37.20 mg KOH/g

Acid number: 0.02 mg KOH/g

Viscosity: 1400 mPa*s, 25 °C

PO/EO/dilactide amount ratios: 17.5%/72.5%/10%

**Polyol 5**

1425.00 g of a 1,2-propane diol (F=2) started polyoxypropylene diol (Polyether 2; Covestro Charge 1712041028) with an OH-number of 233 mg KOH/g, 428.00 g of dilactide (Aldrich, LOT BCBV8611) and 2.00 g of a DMC-catalyst (prepared as stated in WO 01/80994 A1, example 6) were weighed into a 20l stainless-steel pressure reactor under nitrogen atmosphere. After stripping with nitrogen for 30 min. at 100 °C and 0.1 bar, the temperature of the reactor was set to 130 °C. 1929.40 g of propylene oxide (Chemogas) and 6216.90 g of ethylene oxide (GHC Gerling Holz) were dosed within 135 min. to the rection mixture and were reacted for further 45 min at 130°C. Afterwards the reaction was cooled to 90 °C and volatile compounds were distilled off for 30 min. at <0.01 bar. The reaction mixture was stabilized with 6.00 g of Irganox 1076.

Ester group content expressed as -C(=O)-O-: 2.61 wt.-%

Ethylene oxide content: 62.17 wt.-%

OH number: 37.50 mg KOH/g

Acid number: 0.20 mg KOH/g

Viscosity: 802 mPa*s, 25 °C

PO/EO/dilactide amount ratios: 22.5%/72.5%/5%

**Prepolymer Synthesis:**

For the prepolymer synthesis all used polyethers were dehydrated at 120°C and 20 mbar for 2 hours.

**Prepolymer 1**

592.00 g of hexamethylene diisocyanate (HDI) and 0.65 g dibutyl phosphate (DBP) were weighed into a three-neck flask and heated up to 80 °C under constant stirring. 697.50 g of polyether 3 (F=3, OHZ=37, Starter (Glycerin): 1.96%; EO: 70.89%, PO: 26.43%), were added dropwise within 2 hours. After the addition was completed, the reaction was stirred for one additional hour until the residual NCO content reached 21.46%. The reaction mixture was allowed to cool down under constant stirring. Unreacted monomer was removed by thin film evaporation (p=0.1 mbar; $T_{pre-evaporator}$=130 °C, $T_{main-evaporator}$=120 °C) and an NCO-terminated prepolymer with NCO-content= 2.49 wt. % was obtained.

Ester group content expressed as -C(=O)-O-: 0 wt.-%

Residual Monomer content: 0.03 wt.-%

NCO content: 2.49 wt.-%

Viscosity: 3950 mPa*s, 23 °C

Average equivalent weight: 1686.75 g/eq.

**Prepolymer 2**

628.05 g of hexamethylene diisocyanate (HDI) and 0.66 g dibutyl phosphate (DBP) were weighed into a three-neck flask and heated up to 80 °C under constant stirring under nitrogen atmosphere. 700.00 g of polyol 1 were added dropwise within 2 hours. After the addition was completed, the reaction was stirred for one additional hour until residual NCO content reached 22.06%.. The reaction mixture was allowed to cool down under constant stirring. Unreacted monomer was removed by thin film evaporation (p=0.1 mbar; $T_{pre-evaporator}$=130 °C, $T_{main-evaporator}$=120 °C) and an NCO-terminated prepolymer with NCO content= 2.63 wt. % was obtained.

Ester group content expressed as -C(=O)-O-: 0.46 wt.-%

Residual Monomer content: 0.03 wt.-%

NCO content: 2.63 wt.-%

Viscosity: 3967 mPa*s, 23°C

Average equivalent weight: 1596.96 g/eq.

**Prepolymer 3**

625.64 g of hexamethylene diisocyanate (HDI) and 0.66 g dibutyl phosphate (DBP) were weighed into a three-neck flask and heated up to 80 °C under constant stirring under nitrogen atmosphere. 700.00 g of polyol 2, were added dropwise within 2 hours. After the addition was completed, the reaction was stirred for one additional hour until residual NCO content reached 19.41%. The reaction mixture was allowed to cool down under constant stirring. Unreacted monomer was removed by thin film evaporation (p=0.1 mbar; $T_{pre-evaporator}$=130 °C, $T_{main-evaporator}$=120 °C) and an NCO-terminated prepolymer with NCO content= 2.67 wt. % was obtained.

Ester group content expressed as -C(=O)-O-: 1.14 wt.-%

Residual Monomer content: 0.02 wt.-%

NCO content: 2.67 wt.-%

Viscosity: 4451 mPa*s, 23°C

Average equivalent weight: 1573.03 g/eq.

**Prepolymer 4**

592.00 g of hexamethylene diisocyanate (HDI) and 0.61 g dibutyl phosphate (DBP) were weighed into a three-neck flask and heated up to 80 °C under constant stirring. 632.92 g of polyol 3 were added dropwise within 2 hours. After the addition was completed, the reaction was stirred for one additional hour until residual NCO content reached 22.56%. The reaction mixture was allowed to cool down under constant stirring. Unreacted monomer was removed by thin film evaporation (p=0.1 mbar; $T_{pre-evaporator}$=130 °C, $T_{main-evaporator}$=120 °C) and an NCO-terminated prepolymer with NCO content= 2.55 wt. % was obtained.

Ester group content expressed as -C(=O)-O-: 2.29 wt.-%

Residual Monomer content: >0.01 wt.-%

NCO content: 2.53 wt.-%

Viscosity: 3990 mPa*s, 23 °C

Average equivalent weight: 1647.06 g/eq.

**Prepolymer 5**

592.00 g of hexamethylene diisocyanate (HDI) and 0.65 g dibutyl phosphate (DBP) were weighed into a three-neck flask and heated up to 80 °C under constant stirring. 703.92 g of polyol 4 were added dropwise within 2 hours. After the addition was completed, the reaction was stirred for one additional hour until residual NCO content reached 21.30%. The reaction mixture was allowed to cool down under constant stirring. Unreacted monomer was removed by thin film evaporation (p=0.1 mbar; $T_{pre-evaporator}$=130 °C, $T_{main-evaporator}$=120 °C) and an NCO-terminated prepolymer with NCO content= 2.59 wt. % was obtained.

Ester group content expressed as -C(=O)-O-: 4.58 wt.-%

Residual Monomer content: 0.03 wt.-%

NCO content: 2.59 wt.-%

Viscosity: 9451 mPa*s, 23 °C

Average equivalent weight: 1621.62 g/eq.

**Prepolymer 6**

458.80 g of hexamethylene diisocyanate (HDI) and 0.50 g dibutyl phosphate (DBP) were weighed into a three-neck flask and heated up to 80 °C under constant stirring. 541.2 g of polyol 5 were added dropwise within 2 hours. After the addition was completed, the reaction was stirred for one additional hour until residual NCO content reached 21.39%. The reaction mixture was allowed to cool down under constant stirring. Unreacted monomer was removed by thin film evaporation (p=0.1 mbar; $T_{pre-evaporator}$=130 °C, $T_{main-evaporator}$=120 °C) and an NCO-terminated prepolymer with NCO content= 2.23 wt. % was obtained.

Ester group content expressed as -C(=O)-O-: 2.35 wt.-%

Residual Monomer content: 0.02 wt.-%

NCO content: 2.23 wt.-%

Viscosity: 3648 mPa*s, 23 °C

Average equivalent weight: 1883.41 g/eq.

**Table 2: Composition of isocyanate and amine solutions used for the preparation of different hydrogels.**

Comparative examples are denoted as "(c)". Functionalities of the prepolymer and amine are denoted as "F(prep.)" and "F(amine)", respectively. Viscosity is stated in millipascal*seconds and is listed as "[mPa*s]". The solid content of the used isocyanate or amine component is given in weight% and is stated as "[wt.-%]". The molar concentrations of the basic and acidic fractions are denoted as "[M]".

| Ex. | Isocyanate component | | | | Amine component (Jeffamine ED2003) | | | | | | | |
|---|---|---|---|---|---|---|---|---|---|---|---|---|
| | Prepolymer used | F (prep.) | Solid content of the prepared isocyanate solution [wt.-%] | Viscosity of the obtained isocyanate solution at 21 °C [mPa*s] | Amine used | F (amine) | Solid content of the prepared amine solution [wt.-%] | Buffer/Acid type used as solvent | Concentration of basic fraction in the buffer [M] | Concentration of acidic fraction in the buffer [M] | Viscosity of the obtained amine solution at 23 °C [mPa*s] | Molar ratio $NH_2$/NCO |
| 1 (c) | 4 | >2 | 10% | 3.68 | Jeffamine ED2003 | 2 | 11.71% | $H_2O$ | - | - | 2.53 | 1:1 |
| 2a (c) | 1 | >2 | 10% | 3.48 | Jeffamine ED2003 | 2 | 11.71% | Phosphate buffer | 0.0173 | 0.0173 | 2.69 | 1:1 |
| 2b (c) | 1 | >2 | 10% | 3.48 | Jeffamine ED2003 | 2 | 11.71% | Phosphate buffer | 0.02882 | 0.02882 | 3.22 | 1:1 |
| 2c (c) | 1 | >2 | 10% | 3.48 | Jeffamine ED2003 | 2 | 11.71% | Phosphate buffer | 0.0404 | 0.0404 | 3.68 | 1:1 |
| 2d (c) | 1 | >2 | 10% | 3.48 | Jeffamine ED2003 | 2 | 11.71% | Phosphate buffer | 0.0577 | 0.0577 | 2.95 | 1:1 |
| 3a (c) | 1 | >2 | 10% | 3.48 | Jeffamine ED2003 | 2 | 11.71% | Acetate buffer | 0.0173 | 0.0173 | 3.07 | 1:1 |
| 3b (c) | 1 | >2 | 10% | 3.48 | Jeffamine ED2003 | 2 | 11.71% | Acetate buffer | 0.02882 | 0.02882 | 4.22 | 1:1 |
| 3c (c) | 1 | >2 | 10% | 3.48 | Jeffamine ED2003 | 2 | 11.71% | Acetate buffer | 0.0404 | 0.0404 | 3.79 | 1:1 |
| 3d (c) | 1 | >2 | 10% | 3.48 | Jeffamine ED2003 | 2 | 11.71% | Acetate buffer | 0.0669 | 0.0669 | 2.83 | 1:1 |
| 4a (c) | 1 | >2 | 10% | 3.48 | Jeffamine ED2003 | 2 | 11.71% | HCl solution | - | 0.005775 | 2.11 | 1:1 |

Comparative examples are denoted as "(c)". Functionalities of the prepolymer and amine are denoted as "F(prep.)" and "F(amine)", respectively. Viscosity is stated in millipascal*seconds and is listed as "[mPa*s]". The solid content of the used Isocyanate or amine component is given in weight% and is stated as "[wt.-%]". The molar concentrations of the basic and acidic fractions are denoted as "[M]".

| | Isocyanate component | | | | Amine component (Jeffamine ED2003) | | | | | | | |
|---|---|---|---|---|---|---|---|---|---|---|---|---|
| Ex. | Prepolymer used | F (prep.) | Solidcontent of the prepared isocyanate solution [wt.-%] | Viscosity of the obtained isocyanate solution at 21 °C [mPa*s] | Amine used | F (amine) | Solid content of the prepared amine solution [wt.-%] | Buffer/Acid type used as solvent | Concentration of basic fraction in the buffer [M] | Concentration of acidic fraction in the buffer [M] | Viscosity of the obtained amine solution at 23 °C [mPa*s] | Molar ratio NH$_2$/NCO |
| 4b (c) | 1 | >2 | 10% | 3.48 | Jeffamine ED2003 | 2 | 11.71% | HCl solution | - | 0.0173 | 2.35 | 1:1 |
| 4c (c) | 1 | >2 | 10% | 3.48 | Jeffamine ED2003 | 2 | 11.71% | HCl solution | - | 0.02882 | 2.09 | 1:1 |
| 4d (c) | 1 | >2 | 10% | 3.48 | Jeffamine ED2003 | 2 | 11.71% | HCl solution | - | 0.0404 | 2.53 | 1:1 |
| 5 | 5 | > 2 | 11,625% | 4.9 | Jeffamine ED2003 | 2 | 12.20% | Acetate | 0.0299 | 0,0299 | 3.04 | 1:1.2 |
| 6 | 5 | > 2 | 15% | 4.35 | Jeffamine ED2003 | 2 | 15.74% | HCl | - | 0.0276 | 3.58 | 1:1.2 |
| 7 | 5 | > 2 | 15% | 4.35 | Jeffamine ED2003 | 2 | 15.74% | Acetate | 0.0283 | 0.0283 | 4.64 | 1:1.2 |
| 8 | 5 | > 2 | 15% | 4.35 | Jeffamine ED2003 | 2 | 15.74% | HCl | - | 0.0386 | 3.69 | 1:1.2 |
| 9 (c) | 4 | > 2 | 11,625% | 6.56 | Jeffamine ED2003 | 2 | 11.26% | HCl | - | 0.0276 | 3.54 | 1:1.2 |
| 10 | 4 | > 2 | 15% | 8.39 | Jeffamine ED2003 | 2 | 14.53% | HCl | - | 0.0260 | 2.74 | 1:1.2 |
| 11 | 5 | > 2 | 15% | 4.35 | Jeffamine ED2003 | 2 | 15.74% | HCl | - | 0.0213 | 3.82 | 1:1.2 |

| Comparative examples are denoted as "(c)". Functionalities of the prepolymer and amine are denoted as "F(prep.)" and "F(amine)", respectively. Viscosity is stated in millipascal*seconds and is listed as "[mPa*s]". The solid content of the used Isocyanate or amine component is given in weight% and is stated as "[wt.-%]". The molar concentrations of the basic and acidic fractions are denoted as "[M]". |

| | Isocyanate component | | | | Amine component (Jeffamine ED2003) | | | | | | | |
|---|---|---|---|---|---|---|---|---|---|---|---|---|
| Ex. | Prepolymer used | F (prep.) | Solidcontent of the prepared isocyanate solution [wt.-%] | Viscosity of the obtained isocyanate solution at 21 °C [mPa*s] | Amine used | F (amine) | Solid content of the prepared amine solution [wt.-%] | Buffer/Acid type used as solvent | Concentration of basic fraction in the buffer [M] | Concentration of acidic fraction in the buffer [M] | Viscosity of the obtained amine solution at 23 °C [mPa*s] | Molar ratio NH$_2$/NCO |
| 12 | 4 | > 2 | 12.95% | 7.48 | Jeffamine ED2003 | 2 | 12.54% | Acetate | 0.0256 | 0.0256 | 2.34 | 1:1.2 |
| 13 | 4 | > 2 | 15% | 8.39 | Jeffamine ED2003 | 2 | 14.53% | HCl | - | 0.0260 | 3.55 | 1:1.2 |
| 14 (c) | 5 | >2 | 10% | 3.30 | Jeffamine ED2003 | 2 | 10.50% | HCl | - | 0.0257 | 2.65 | 1:1.2 |
| 15 (c) | 5 | >2 | 10% | 3.30 | Jeffamine ED2003 | 2 | 10.50% | Acetate | 0.0154 | 0.0154 | 3.37 | 1:1.2 |
| 16 | 4 | >2 | 15% | 8.39 | Jeffamine ED2003 | 2 | 14.53% | Acetate | 0.0356 | 0.0356 | 4.65 | 1:1.2 |
| 17 | 5 | >2 | 12.787% | 4.48 | Jeffamine ED2003 | 2 | 13.43% | Acetate | 0.0197 | 0.0197 | 3.47 | 1:1.2 |
| 18 | 4 | >2 | 13.375% | 8.51 | Jeffamine ED2003 | 2 | 12.95% | Acetate | 0.0191 | 0.0191 | 2.47 | 1:1.2 |
| 19 | 5 | >2 | 14.25% | 6.82 | Jeffamine ED2003 | 2 | 14.96% | Acetate | 0.0346 | 0.0346 | 4.85 | 1:1.2 |
| 20 (c) | 2 | >2 | 10% | 3.80 | Jeffamine ED2003 | 2 | 10.66% | HCl | - | 0,02609 | 2.64 | 1:1.2 |
| 21 | 3 | >2 | 12.5% | 4.82 | Jeffamine ED2003 | 2 | 13.52% | HCl | - | 0,01987 | 3.26 | 1:1.2 |

| Comparative examples are denoted as "(c)". Functionalities of the prepolymer and amine are denoted as "F(prep.)" and "F(amine)", respectively. Viscosity is stated in millipascal*seconds and is listed as "[mPa*s]". The solid content of the used Isocyanate or amine component is given in weight% and is stated as "[wt.-%]". The molar concentrations of the basic and acidic fractions are denoted as "[M]". |||||||||||||
|---|---|---|---|---|---|---|---|---|---|---|---|---|
| | Isocyanate component | | | | Amine component (Jeffamine ED2003) | | | | | | | |
| Ex. | Prepolymer used | F (prep.) | Solidcontent of the prepared isocyanate solution [wt.-%] | Viscosity of the obtained isocyanate solution at 21 °C [mPa*s] | Amine used | F (amine) | Solid content of the prepared amine solution [wt.-%] | Buffer/Acid type used as solvent | Concent ration of basic fraction in the buffer [M] | Concentra tion of acidic fraction in the buffer [M] | Viscosity of the obtained amine solution at 23 °C [mPa*s] | Molar ratio $NH_2$/ NCO |
| 22 (c) | 3 | >2 | 10% | 3.11 | Jeffamine ED2003 | 2 | 10.82% | Acetate | 0,02649 | 0,02649 | 2.42 | 1:1.2 |
| 23 | 3 | > 2 | 11.25% | 4.51 | Jeffamine ED2003 | 2 | 12.17% | Acetate | 0,02384 | 0,02384 | 2.57 | 1:1.2 |
| 24 (c) | 2 | > 2 | 15% | 7.21 | Jeffamine ED2003 | 2 | 15.98% | Acetate | 0,02348 | 0,02348 | 4.88 | 1:1.2 |
| 25 (c) | 3 | >2 | 10% | 3.11 | Jeffamine ED2003 | 2 | 10.82% | Acetate | 0,02649 | 0,02649 | 2.35 | 1:1.2 |
| 26 (c) | 3 | >2 | 10% | 3.11 | Jeffamine ED2003 | 2 | 10.82% | HCl | - | 0,02119 | 2.57 | 1:1.2 |
| 27 (c) | 2 | >2 | 15% | 7.21 | Jeffamine ED2003 | 2 | 15.98% | Acetate | 0,02348 | 0,02348 | 4.12 | 1:1.2 |
| 28 (c) | 2 | > 2 | 12.5% | 4.87 | Jeffamine ED2003 | 2 | 13.32% | HCl | - | 0,01957 | 3.19 | 1:1.2 |
| 29 (c) | 2 | > 2 | 15% | 7.21 | Jeffamine ED2003 | 2 | 15.98% | HCl | - | 0,03914 | 3.68 | 1:1.2 |
| 30 (c) | 2 | >2 | 10% | 3.80 | Jeffamine ED2003 | 2 | 10.66% | HCl | - | 0,02087 | 2.68 | 1:1.2 |
| 31 (c) | 6 | 2 | 10.8% | 3.74 | Jeffamine T403 | 3 | 1.55% | Acetate | 0.0265 | 0.0265 | 1.11 | 1:1.2 |

(continued)

| | Isocyanate component | | | | Amine component (Jeffamine ED2003) | | | | | | | |
|---|---|---|---|---|---|---|---|---|---|---|---|---|
| Ex. | Prepolymer used | F (prep.) | Solid content of the prepared isocyanate solution [wt.-%] | Viscosity of the obtained isocyanate solution at 21 °C [mPa*s] | Amine used | F (amine) | Solid content of the prepared amine solution [wt.-%] | Buffer/Acid type used as solvent | Concentration of basic fraction in the buffer [M] | Concentration of acidic fraction in the buffer [M] | Viscosity of the obtained amine solution at 23 °C [mPa*s] | Molar ratio $NH_2$/ NCO |
| 32 (c) | 6 | 2 | 13.5% | 4.72 | Jeffamine T403 | 3 | 1.94% | HCl | - | 0.020 | 1.18 | 1:1.2 |
| 33 | 6 | 2 | 16.25% | 6.97 | Jeffamine T403 | 3 | 2.33% | $H_2O$ | - | - | 1.30 | 1:1.2 |
| 34 (c) | 6 | 2 | 11.17% | 3.74 | Jeffamine ED2003 | 2 | 12.11% | Acetate | 0.01857 | 0.01857 | 2.81 | 1:1 |

**Table 3: Solid content of the obtained hydrogels based on formulations given in table 3.**

Comparative examples are denoted as "(c)".

| Example | 1 (c) | 2a (c) | 2b (c) | 2c (c) | 2d (c) | 3a (c) | 3b (c) | 3c (c) | 3d (c) | 4a (c) |
|---|---|---|---|---|---|---|---|---|---|---|
| Solids content [weight-%] | 10.57 | 10.57 | 10.57 | 10.57 | 10.57 | 10.57 | 10.57 | 10.57 | 10.57 | 10.57 |
| Example | 4b (c) | 4c (c) | 4d (c) | 5 | 6 | 7 | 8 | 9 (c) | 10 | 11 |
| Solids content [weight-%] | 10.57 | 10.57 | 10.57 | 11.73 | 14.74 | 14.74 | 14.74 | 11.50 | 14.84 | 14.74 |
| Example | 12 | 13 | 14 (c) | 15 (c) | 16 | 17 | 18 | 19 | 20 (c) | 21 |
| Solids content [weight-%] | 12.81 | 14.84 | 10.09 | 10.16 | 14.84 | 13.00 | 13.23 | 14.49 | 10.22 | 12.84 |
| Example | 22 (c) | 23 | 24 (c) | 25 (c) | 26 (c) | 27 (c) | 28 (c) | 29 (c) | 30 (c) | 31 (c) |
| Solids content [weight-%] | 10.27 | 11.56 | 15.33 | 10.27 | 10.27 | 15.33 | 12.77 | 15.33 | 10.22 | 7.72 |
| Example | 32 (c) | 33 | 34 (c) | | | | | | | |
| Solids content [weight-%] | 9.65 | 11.61 | 11.48 | | | | | | | |

**Table 4: Overview on results of important parameters for an adhesion prevention.**

Comparative examples are denoted as "(c)" and "n. a." denotes that the property in question has not been analyzed. For the purposes of the present invention the following thresholds were applied to denote the suitability of the hydrogel: loss of mechanical properties: < 28 days; adhesion on organs: 2; 3; 4 or 5; curing time: < 120 seconds.

The adhesion on organs was rated on a scale of 1 to 5 according to the score previously discussed with 1 denoting the poorest and 5 the best adhesion.

| Example | pH after 30 min. | Loss of mechanical properties [d] | Elongation [%] | Tensile strength [kPa] | Adhesion on organs | Curing time [s] |
|---|---|---|---|---|---|---|
| 1 (c) | 9.84 | n. a. | n. a. | n. a. | 1 | 15 |
| 2a (c) | 8.72 | n. a. | n. a. | n. a. | 1 | 20 |
| 2b (c) | 8.14 | n. a. | n. a. | n. a. | 1 | 20 |
| 2c (c) | 7.84 | n. a. | n. a. | n. a. | 1 | 20 |
| 2d (c) | 7.51 | n. a. | n. a. | n. a. | 1 | 120 |
| 3a (c) | 8.61 | n. a. | n. a. | n. a. | 1 | 20 |
| 3b (c) | 8.9 | n. a. | n. a. | n. a. | 1 | 20 |
| 3c (c) | 6.65 | n. a. | n. a. | n. a. | 1 | 150 |
| 3d (c) | 6.46 | n. a. | n. a. | n. a. | 1 | No curing observed |
| 4a (c) | 9.44 | n. a. | n. a. | n. a. | 1 | 20 |
| 4b (c) | 6.76 | n. a. | n. a. | n. a. | 1 | 20 |
| 4c (c) | 6.78 | n. a. | n. a. | n. a. | 1 | 20 |
| 4d (c) | 6.71 | n. a. | n. a. | n. a. | 1 | 20 |
| 5 | 7.3 | 0.33 | 128 | 11.27 | 2 | 29 |
| 6 | 6.65 | 3 | 89 | 12.78 | 5 | 24 |

(continued)

| Example | pH after 30 min. | Loss of mechanical properties [d] | Elongation [%] | Tensile strength [kPa] | Adhesion on organs | Curing time [s] |
|---|---|---|---|---|---|---|
| 7 | 6.82 | 6 | 70 | 16.69 | 5 | 19 |
| 8 | 6.7 | 3 | 116 | 12.87 | 4 | 18 |
| 9 (c) | 6.65 | 7 | 258 | 32.33 | 1 | 24 |
| 10 | 7.86 | 13 | 190 | 32.9 | 4 | 11 |
| 11 | 6.92 | 3 | 174 | 43.2 | 4 | 18 |
| 12 | 7.08 | 8 | 278 | 92.5 | 2 | 13 |
| 13 | 6.59 | 13 | 256 | 63.4 | 4 | 9 |
| 14 (c) | 6.7 | 2 | 257 | 57.2 | 1 | 64 |
| 15 (c) | 7.47 | 1.33 | 88 | 7.7 | 1 | 50 |
| 16 | 6.52 | 9 | 319 | 72.6 | 4 | 22 |
| 17 | 7.81 | 6 | 121 | 28.6 | 3 | 27 |
| 18 | 6.74 | 12 | 214 | 67.9 | 4 | 9 |
| 19 | 7.17 | 6 | 96 | 17.3 | 5 | 12 |
| 20 (c) | 6.67 | 1 | 311.14 | 15.52 | 1 | 360 |
| 21 | 6.7 | 11 | 143.2 | 34.68 | 2 | 16 |
| 22 (c) | 6.62 | 10 | 273.75 | 62.27 | 1 | 30 |
| 23 | 6.63 | 10 | 223.65 | 18.13 | 3 | 17 |
| 24 (c) | 6.76 | >28 | 243.36 | 61.71 | 5 | 7 |
| 25 (c) | 6.74 | 10 | 191.07 | 13.62 | 1 | 29 |
| 26 (c) | 6.72 | 9 | 239.39 | 20.82 | 1 | 22 |
| 27 (c) | 6.73 | >28 | 169.81 | 39.39 | 4 | 7 |
| 28 (c) | 6.69 | >28 | 273.48 | 44.44 | 5 | 13 |
| 29 (c) | 6.88 | >28 | 380.56 | 52.3 | 5 | 9 |
| 30 (c) | 6.63 | >28 | 172.12 | 16.56 | 1 | 30 |
| 31 (c) | n. a. | n. a. | n. a. | n. a. | n. a. | No curing observed |
| 32 (c) | n. a. | n. a. | n. a. | n. a. | n. a. | No curing observed |
| 33 | n. a. | n. a. | 240.96 | 41.58 | n. a. | 23 |
| 34 (c) | n. a. | n. a. | n. a. | n. a. | n. a. | No curing observed |

**Claims**

1.  A method of manufacturing a hydrogel comprising reacting the components:

    A) An isocyanate-terminated prepolymer obtained by reacting a mixture comprising:

    A1) A diisocyanate with a molar weight of 140 to 278 g/mol;

A2) A polyalkylene oxide with an ethylene oxide content of ≥ 50 weight-%, calculated from the total weight of the polyalkylene oxide;

B) A polyamine having a molecular weight of ≥ 200 g/mol;
C) Water;

**characterized in that**

the polyalkylene oxide A2) or the polyamine B) further comprise ester groups, expressed as -C(=O)-O- groups, in an amount of ≥ 0.51 weight-%, based on the total weight of polyalkylene oxide A2) or polyamine B), respectively, the average OH functionality of polyalkylene oxide A2) is greater than 2 and/or the average combined primary and secondary amine functionality of polyamine B) is greater than 2,
the combined components A) and B) are present in an amount of ≥ 11.5 weight-%, based on the total weight of components A), B) and C).

2. The method according to claim 1, wherein the NCO/NH molar ratio between prepolymer A) and polyamine B) is > 1.0 to ≤ 1.4.

3. The method according to claim 1 or 2, wherein the prepolymer A) and/or the polyamine B) are provided as aqueous solutions.

4. The method according to any one of the preceding claims, wherein the aqueous solution of prepolymer A) and/or the aqueous solution of polyamine B) have a viscosity of ≤ 50 mPas at 23 °C as determined according to DIN EN ISO 3219.

5. The method according to one of the preceding claims, wherein the water C) comprises an acid or a pH buffer.

6. The method according to one of the preceding claims, wherein the diisocyanate A1) is an aliphatic diisocyanate.

7. The method according to one of the preceding claims, wherein the prepolymer A) has an NCO group content of ≥ 1 weight-% to ≤ 10 weight-% as determined according to DIN EN ISO 11909.

8. The method according to one of the preceding claims, wherein the polyamine B) is an amino group-terminated polyether.

9. The method according to one of the preceding claims, wherein the equivalent weights of the prepolymer A) and the polyamine B) have a ratio of ≥ 0.25 to ≤ 4.

10. The method according to one of claims 3 to 9, wherein aqueous solutions of prepolymer A) and polyamine B) are mixed in a mixing or spraying head and the resulting mixture is applied onto a target surface, thereby forming the hydrogel on the target surface.

11. The method according to one of the preceding claims, further comprising adding as component D) an active pharmaceutical ingredient to a precursor of the hydrogel and/or to the hydrogel itself.

12. A hydrogel obtainable by a method according to one of claims 1 to 11.

13. A hydrogel obtainable by a method according to one of claims 1 to 11 for use in a method of surgery.

14. A hydrogel obtainable by a method according to one of claims 1 to 11 for use as a post-surgical adhesion barrier.

15. A device comprising a first storage volume, a second storage volume and a mixer in fluid communication with the first and second storage volume, wherein the mixer is configured to mix the contents of the first and second storage volume and to discharge the resulting mixture from the device,
**characterized in that**
the first storage volume comprises the prepolymer A) as recited in claim 1 or 7, the second storage volume comprises the polyamine B) as recited in claim 1 or 8.

Europäisches
Patentamt

European
Patent Office

Office européen
des brevets

# EUROPEAN SEARCH REPORT

**Application Number**

**EP 23 15 4695**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | DE 20 2018 004305 U1 (COVESTRO DEUTSCHLAND AG [DE]) 30 October 2018 (2018-10-30) * paragraphs [0001], [0002], [0006] – [0019], [0023] – [0026], [0037] – [0039] * * paragraphs [0041] – [0050] * | 1-15 | INV. A61L26/00 C08G18/10 C08G18/16 C08G18/48 C08G18/50 C08G18/73 |
| A,D | US 2012/244107 A1 (HECKROTH HEIKE [DE] ET AL) 27 September 2012 (2012-09-27) * paragraphs [0001], [0002], [7/23], [0052] – [0073] * * paragraphs [0185], [0186]; examples 1-20,33 * | 1-15 | |
| A | DATABASE WPI Week 201582 Thomson Scientific, London, GB; AN 2015-616646 XP002809463, & CN 104 861 176 A (CAS QINGDAO BIOMASS ENERGY & BIOPROCESS TEC) 26 August 2015 (2015-08-26) * abstract * | 1-15 | TECHNICAL FIELDS SEARCHED (IPC) C08G A61L |
| A | US 2009/191145 A1 (HECKROTH HEIKE [DE] ET AL) 30 July 2009 (2009-07-30) * paragraphs [0001], [0014] – [0030] * * claims 13,14; examples 1-3a * | 1-15 | |

The present search report has been drawn up for all claims

| Place of search | Date of completion of the search | Examiner |
|---|---|---|
| Munich | 22 June 2023 | Neugebauer, Ute |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 4695

22-06-2023

| Patent document cited in search report | Publication date | Patent family member(s) | Publication date |
|---|---|---|---|
| DE 202018004305 U1 | 30-10-2018 | NONE | |
| US 2012244107 A1 | 27-09-2012 | AU 2010310208 A1 | 10-05-2012 |
| | | BR 112012009266 A2 | 31-05-2016 |
| | | CA 2780919 A1 | 28-04-2011 |
| | | CN 102573947 A | 11-07-2012 |
| | | EP 2490728 A1 | 29-08-2012 |
| | | JP 2013508482 A | 07-03-2013 |
| | | KR 20120098638 A | 05-09-2012 |
| | | US 2012244107 A1 | 27-09-2012 |
| | | WO 2011047789 A1 | 28-04-2011 |
| CN 104861176 A | 26-08-2015 | NONE | |
| US 2009191145 A1 | 30-07-2009 | AU 2009207883 A1 | 30-07-2009 |
| | | BR PI0906578 A2 | 07-07-2015 |
| | | CA 2712648 A1 | 30-07-2009 |
| | | CN 101925625 A | 22-12-2010 |
| | | EP 2083025 A1 | 29-07-2009 |
| | | EP 2245078 A1 | 03-11-2010 |
| | | ES 2396020 T3 | 18-02-2013 |
| | | JP 2011511857 A | 14-04-2011 |
| | | KR 20100117573 A | 03-11-2010 |
| | | TW 200948839 A | 01-12-2009 |
| | | US 2009191145 A1 | 30-07-2009 |
| | | WO 2009092524 A1 | 30-07-2009 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 202018004305 U1
- US 2012244107 A1
- US 4795764 A
- WO 2003050276 A1
- US 2013060216 A1
- US 2013204217 A1
- WO 0180994 A1